# EUROPEAN PATENT SPECIFICATION

(11) **EP 3 996 641 B1**
(45) Date of publication and mention of the grant of the patent: **11.10.2023**
(21) Application number: 20750875.5
(22) Date of filing: 06.07.2020
(51) Int. Cl.: A61B 10/00, A61F 5/44, A61F 5/451

(54) **FLUID COLLECTION DEVICES INCLUDING AN OPENING HAVING AN INCREASED WIDTH**
FLÜSSIGKEITSSAMMELVORRICHTUNG MIT EINER ÖFFNUNG MIT ERHÖHTER BREITE
DISPOSITIFS DE COLLECTE DE FLUIDE COMPRENANT UNE OUVERTURE AYANT UNE LARGEUR ACCRUE

(30) Priority: 09.07.2019 US 201962871830 P
(43) Date of publication of application: 18.05.2022
(73) Proprietor: Purewick Corporation, El Cajon, California 92020 (US)
(72) Inventor: JOHANNES, Ashley, Marie, Atlanta, GA 30340 (US); SKELTON, Sarah, Atlanta, GA 30312 (US); EVANS, Megan, Grove City, OH 43123 (US)
(74) Representative: Hoffmann Eitle
(86) International application number: PCT/US2020/040860
(87) International publication number: WO 2021/007144

(56) References cited:
- WO-A2-2011/087871
- WO-A2-2014/126888
- US-A- 4 747 166
- US-A1- 2017 266 031

## Description

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, pressure ulcers spills, and other hygiene issues. Urinary catheters be may be uncomfortable, painful, and may cause urinary tract infections.

US 2017/0266031 A1 discloses an elongate wearable, urine-collecting assembly having a fluid impermeable casing with a urine reservoir at one end and a fluid outlet at the other end and, in between, a longitudinally elongate opening through which urine from the wearer enters the casing to be collected in the reservoir and advanced out of the casing through the outlet. The casing is substantially cylindrical.

US 4,747,166 discloses a wearable urine collection device that comprises an elongate urine-absorptive pad which receives and retains urine. Urine is aspirated out of the pad through a multitude of perforations in the wall of a vacuum tube that are present throughout the length of the tube within the pad.

Thus, users and manufacturers of fluid collection devices continue to seek new and improved devices, systems, and methods to collect urine.

### SUMMARY

The invention is defined in claim 1 and the dependent claims are directed to preferred embodiments and optional features. The pre-characterizing part of claim 1 corresponds to the disclosure of US 2017/266031 A1. Disclosed herein are fluid collection devices and methods of assembling fluid collection devices. In an embodiment, a fluid collection device includes a fluid impermeable barrier and a fluid permeable body. The fluid impermeable barrier at least partially defines a chamber, an aperture configured to receive a conduit therethrough, and an opening. The fluid permeable body is positioned at least partially within the chamber to extend across at least a portion of the opening and configured to wick fluid away from the opening. The fluid permeable body includes a slot extending longitudinally through the fluid permeable body and configured to at least partially receive a conduit therein.

In an embodiment, a fluid collection device includes a fluid impermeable barrier and a fluid permeable body. The fluid impermeable barrier has a first lateral side defining an aperture configured to receive a conduit therethrough, a second lateral side distal to the first lateral side, and a protrusion extending from the second lateral side. The fluid impermeable barrier also defines a chamber and an opening. The fluid permeable body is positioned at least partially within the chamber to extend across at least a portion of the opening and to at least partially define, with the fluid impermeable barrier, a reservoir that is positioned in the protrusion of the fluid impermeable barrier. The fluid permeable body is configured to wick fluid away from the opening to the reservoir. The opening includes a longitudinal length of at least about 4 cm and a lateral width that is substantially equal to or greater than the longitudinal length. The fluid permeable body being configured to wick fluid away from the opening to the reservoir.

In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a front plan view of a fluid collection device, according to a first embodiment.
**FIG. 2A** is a cross-sectional view of the fluid collection device of **FIG. 1** taken along line 2-2 thereof.
**FIG. 2B** is a cross-sectional view of the fluid collection device of **FIG. 1** taken along line 2-2 thereof and with the conduit removed.
**FIG. 3** is a cross-sectional view of the fluid collection device of **FIG. 1** taken along line 3-3 thereof.
**FIG. 4** is a cross-section view of a fluid impermeable barrier of the fluid collection device of **FIG. 1** taken along line 3-3 thereof with a fluid permeable body and conduit removed.
**FIG. 5** is a flow diagram of a method of assembling a fluid collection device.
**FIG. 6** is a flow diagram of a method to collect fluid.
**FIG. 7** is a block diagram of a system for fluid collection.

### DETAILED DESCRIPTION

Disclosed herein are urine collection devices. Proper positioning of a urine collection device on a user is an important factor in the collection device collecting urine from the user. Certain physical attributes of users, however, often make proper positioning of the collection device difficult to maintain. For example, in a female having a thinner physique, it may be difficult for a cylindrical urine collection device to remain positioned between the legs of the female and against the urethra of the female because the user does not have the flesh necessary to hold the collection device in place. In another example, a male may have a buried penis that is covered by excess skin in the pubic area or scrotum, making it difficult to place a collection device directly against the urethral of the male. Embodiments of the collection devices disclosed herein include collection devices that are substantially flat and wider than conventional urine collection devices. A substantially flat, wider urine collection device allows for easier positioning and securement of the collection device on thinner patients. For example, a substantially flat, wider urine fluid collection device, in some embodiments, uses the inner thighs or seam between the thighs and pelvis for more secure positioning of the urine collection device against the urethra of a female user. In another example of a man having a buried penis, a substantially flat, wider urine collection device may be placed on the skin of the male over the buried penis. The wider fluid collection device covers more surface area on the male to allow the collection device to collect more of the discharged urine from folds in the skin leading to the buried penis. The collection device includes a fluid impermeable barrier and a fluid permeable body. The fluid impermeable barrier at least partially defines a chamber, an aperture configured to receive a conduit therethrough, and an opening. The fluid permeable body is positioned at least partially within the chamber to extend across at least a portion of the opening and configured to wick fluid away from the opening. The fluid permeable body includes a slot extending longitudinally through the fluid permeable body and configured to at least partially receive a conduit therein.

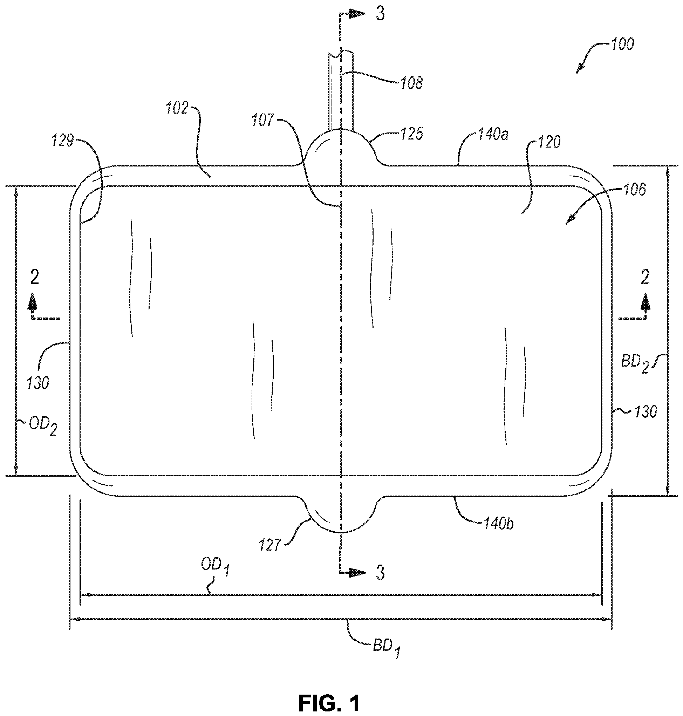

**FIG. 1** is a front plan view of a urine collection device 100, according to an embodiment. The fluid collection device 100 is an example of a fluid collection device 100 that is configured to receive fluids from either a female or male. The fluid collection device 100 includes a fluid impermeable barrier 102 having a lateral width BD₁ that is substantially equal to or greater than a longitudinal length BD₂. With a lateral width that is substantially equal to or greater than a longitudinal length, the fluid impermeable barrier 102 may include various general geometric front profiles, such as substantially rectangular, substantially diamond shaped, substantially circular, substantially oval, and so forth. For example, the fluid impermeable barrier 102 shown in FIG. 1 includes a substantially rectangular front profile having a first lateral side 140a, a second lateral side 140b distal to the first lateral side 140a, and two longitudinal sides 130 each extending between the first lateral side 140a and the second lateral side 140b. The first lateral side 140a and the second lateral side 140b may each have a lateral width BD₁, and the two longitudinal sides 130 may each have a longitudinal length BD₂.

In many embodiments, the lateral width BD₁ of the fluid permeable barrier 102 is greater than the longitudinal length BD₂ of the fluid impermeable barrier 102. For example, the lateral width BD₁ may be at least about 1.25 times greater than the longitudinal length BD₂, at least about 1.5 times greater than the longitudinal length BD₂, at least about 1.75 times greater than the longitudinal length BD₂, at least about 2 times greater than the longitudinal length BD₂, at least about 2.25 times greater than the longitudinal length BD₂, at least about 2.5 times greater than the longitudinal length BD₂, at least about 2.75 times greater than the longitudinal length BD₂, at least about 3 times greater than the longitudinal length BD₂, about 1.25 to about 3.25 times greater than the longitudinal length BD₂, about 1.25 to about 2.25 times greater than the longitudinal length BD₂, about 2.25 to about 3.25 times greater than the longitudinal length BD₂, about 1.25 to about 1.75 times greater than the longitudinal length BD₂, about 1.75 to about 2.25 times greater than the longitudinal length BD₂, about 2.25 to about 2.75 time greater than the longitudinal length BD₂, or about 2.75 to about 3.25 times greater than the longitudinal length BD₂. The lateral width BD₁ may vary according to different embodiments. For example, the lateral width BD₁ may be about 1.5 inches (about 3.8 cm) to about 5 inches (about 12.7 cm), about 1.5 inches to about 4 inches (about 10.2 cm), about 1.5 inches to about 3 inches (about 7.6 cm), about 1.5 inches to about 2.5 inches (about 6.4 cm), about 2 inches (about 5.1 cm) to about 3 inches, about 2.5 inches to about 3.5 inches (about 8.9 cm), about 3 inches to about 4 inches, about 3.5 inches to about 4.5 inches (about 11.4 cm), about 4 inches to about 5 inches, about 1.5 to about 2 inches, about 2 inches to about 2.5 inches, about 2.5 inches to about 3 inches, about 3 inches to about 3.5 inches, about 3.5 inches to about 4 inches, about 4 inches to about 4.5 inches, about 4.5 inches about 5 inches, at least about 1.5 inches, at least about 2 inches, at least about 2.5 inches, at least about 3 inches, at least about 3.5 inches, at least about 4 inches, at least about 4.5 inches, at least about 5 inches, about 1.5 inches, about 2 inches, about 2.5 inches, about 3 inches, about 3.5 inches, about 4 inches, about 4.5 inches, about 5 inches, less than about 1.5 inches, less than about 2 inches, less than about 2.5 inches, less than about 3 inches, less than about 3.5 inches, less than about 4 inches, less than about 4.5 inches, or less than about 5 inches.

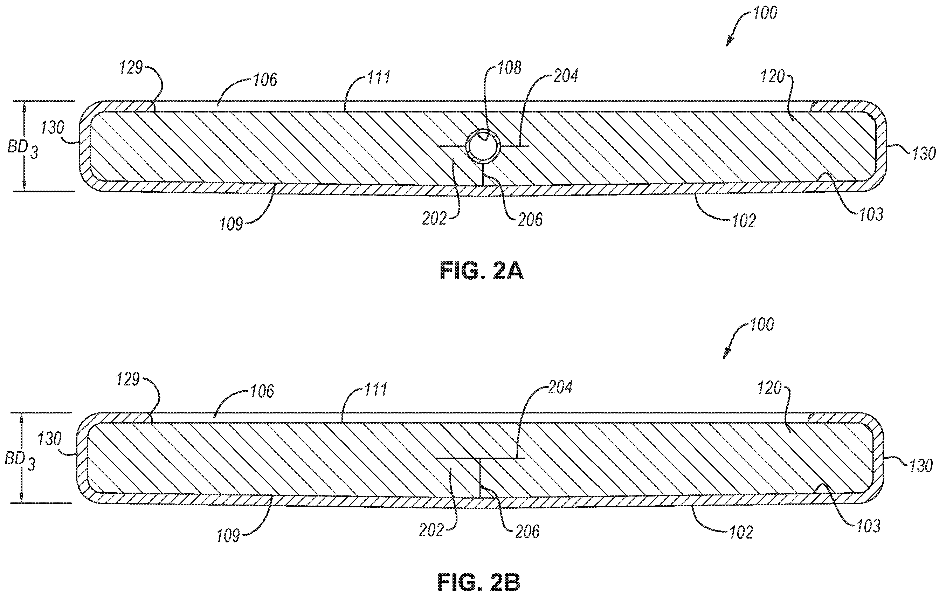

Whether the fluid impermeable barrier 102 includes a substantially rectangular, substantially diamond-shaped, substantially circular, or substantially oval front profile, the fluid impermeable barrier 102 is substantially flat or planar (as shown in **FIGS. 2A-4**). That is, the fluid impermeable barrier 102 may include a substantially flat back side and define an opening 106 on the front side having a substantially planar profile. The depth of the fluid impermeable barrier 102 may vary according to different embodiments. For example, the fluid impermeable barrier 102 may include a depth BD₃ (shown in **FIGS. 2A-2B**) of about 0.25 inch (about 6.4 cm) to about 0.5 inch (about 1.3 cm), 0.5 inch to about 0.75 inch (about 1.9 cm), 0.75 inch to about 1 inch, 0.25 inch to about 0.33 inch (about 0.84 cm), about 0.33 inch to about 0.5 inch, about 0.5 inch to about 0.66 inch (about 1.68 cm), about 0.66 inch to about 0.75 inch, at least about 0.25 inch, at least about 0.33 inch, at least about 0.5 inch, at least about 0.66 inch, at least about 0.75 inch, about 0.25 inch, about 0.33 inch, about 0.5 inch, about 0.66 inch, about 0.75 inch, less than about 0.25 inch, less than about 0.33 inch, less than about 0.5 inch, less than about 0.66 inch, or less than about 0.75 inch. The depth BD₃ is less than both the lateral width BD₁ and the longitudinal length BD₂. For example, the longitudinal length BD₂ may be at least 1.25 times greater than the depth BD₃, at least 1.5 times greater than the depth BD₃, at least 1.75 times greater than the depth BD₃, at least 2 times greater than the depth BD₃, at least 2.5 times greater than the depth BD₃, or at least 3 times greater than the depth BD₃.

The fluid impermeable barrier 102 also includes one or more protrusions extending from a side of the fluid impermeable barrier 102. More specifically, the fluid impermeable barrier 102 shown in **FIG. 1** includes a protrusion 127 extending longitudinally from the lateral side 140b. The protrusion 127 may extend from the lateral side 140b at least about 0.25 inch, at least about 0.5 inch, at least about 0.75 inch, at least about 1 inch, at least about 1.25 inches (about 3.2 cm), at least about 1.5 inches, at least about 1.75 inches (about 4.4 cm) , at least about 2 inches, about 0.25 inch to about 2 inches, about 0.25 inch to about 1 inch, about 1 inch to about 2 inches, about 0.25 inch to about 0.75 inch, about 0.5 inch to about 1 inch, about 0.75 inch to about 1.25 inches, about 1 inch to about 1.5 inches, about 1.25 inches to about 1.75 inches, or about 1.5 inches to about 2 inches. The protrusion 127 may include a maximum lateral width of at least about 0.5 inch, at least about 0.75 inch, at least about 1 inch, at least about 1.25 inches, about 0.25 inch to about 2 inches, about 0.25 inch to about 1.25 inches, about 0.25 inch to about 0.5 inch, about 0.5 inch to about 0.75 inch, about 0.75 inch to about 1 inch, or about 1 inch to about 1.25 inches, about 1.25 inches to about 1.75 inches, or about 1.5 inches to about 2 inches. The protrusion 127 may include any combination of extending dimensions and lateral widths provided above.

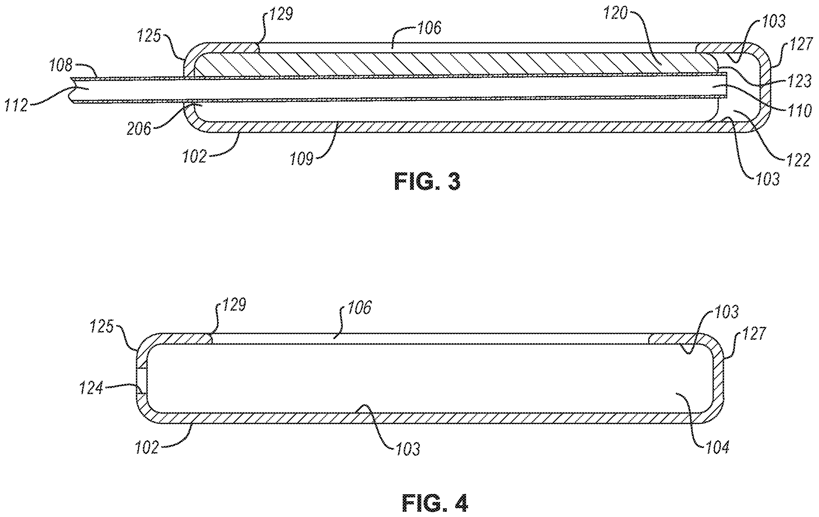

The protrusion 127 may be a bulbous, semispherical, or cylindrical protrusion extending from the lateral side 140b. The protrusion 127 also may include two portions of the lateral side 140b sloping towards an apex of the protrusion 127. The protrusion 127 at least partially defines a reservoir 122 (shown in **FIG. 3**) within the chamber 104, described in greater detail below. The protrusion 127 is positioned on an axis 107 that is substantially centered on a lateral width OD₁ of the opening 106 or the lateral width BD₁ of the fluid impermeable barrier 102. The fluid impermeable barrier 102 also may include an additional protrusion 125 positioned opposite to the protrusion 127, such as extending longitudinally from the lateral side 140a. The protrusion 125 defines an aperture 124 (shown in **FIG. 4**) sized to receive a conduit 108 (*e.g.,* at least one tube). The aperture 124 is positioned on the axis 107 that is substantially centered on the lateral width OD₁ of the opening 106 or the lateral width BD₁ of the fluid impermeable barrier 102. The aperture 124 may be configured to form an at least substantially fluid tight seal against the conduit 108 or the at least one tube thereby substantially preventing the fluids from escaping the chamber 104.

The fluid impermeable barrier 102 also at least partially defines a chamber 104 (*e.g.,* interior region, shown in **FIG. 4**) and includes an inward border or edge 129 defining the opening 106. The opening 106 includes a longitudinal length OD₂ and a lateral width OD₁ that is substantially equal to or greater than the longitudinal length OD₂. With a lateral width OD₁ that is substantially equal to or greater than a longitudinal length OD₂, the opening 106 may include various general geometric front profiles, such as substantially rectangular, substantially diamond shaped, substantially circular, substantially oval, and so forth. For example, the opening 106 shown in **FIG. 1** includes a substantially rectangular opening 106 having a lateral width OD₁ and a longitudinal length OD₂.

In many embodiments, the lateral width OD₁ of the opening 106 is greater than the longitudinal length OD₂ of the opening 106. For example, the lateral width OD₁ may be at least about 1.25 times greater than the longitudinal length OD₂, at least about 1.5 times greater than the longitudinal length OD₂, at least about 1.75 times greater than the longitudinal length OD₂, at least about 2 times greater than the longitudinal length OD₂, at least about 2.25 times greater than the longitudinal length OD₂, at least about 2.5 times greater than the longitudinal length OD₂, at least about 2.75 times greater than the longitudinal length OD₂, at least about 3 times greater than the longitudinal length OD₂, about 1.25 to about 3.25 times greater than the longitudinal length OD₂, about 1.25 to about 2.25 times greater than the longitudinal length OD₂, about 2.25 to about 3.25 times greater than the longitudinal length OD₂, about 1.25 to about 1.75 times greater than the longitudinal length OD₂, about 1.75 to about 2.25 times greater than the longitudinal length OD₂, about 2.25 to about 2.75 time greater than the longitudinal length OD₂, or about 2.75 to about 3.25 times greater than the longitudinal length OD₂. The lateral width OD₁ may vary according to different embodiments. For example, the lateral width OD₁ may be about 1.5 inches to about 5 inches, about 1.5 inches to about 4 inches, about 1.5 inches to about 3 inches, about 1.5 inches to about 2.5 inches, about 2 inches to about 3 inches, about 2.5 inches to about 3.5 inches, about 3 inches to about 4 inches, about 3.5 inches to about 4.5 inches, about 4 inches to about 5 inches, about 1.5 to about 2 inches, about 2 inches to about 2.5 inches, about 2.5 inches to about 3 inches, about 3 inches to about 3.5 inches, about 3.5 inches to about 4 inches, about 4 inches to about 4.5 inches, about 4.5 inches about 5 inches, at least about 1.5 inches, at least about 2 inches, at least about 2.5 inches, at least about 3 inches, at least about 3.5 inches, at least about 4 inches, at least about 4.5 inches, at least about 5 inches, about 1.5 inches, about 2 inches, about 2.5 inches, about 3 inches, about 3.5 inches, about 4 inches, about 4.5 inches, about 5 inches, less than about 1.5 inches, less than about 2 inches, less than about 2.5 inches, less than about 3 inches, less than about 3.5 inches, less than about 4 inches, less than about 4.5 inches, or less than about 5 inches.

The fluid impermeable barrier 102 may form a lip surrounding at least a portion of the opening 106. The lip surrounding at least a portion of the opening may be about one-half the difference between BD₁ and OD₁ or BD₂ and OD₂. BD₁ may be greater than OD₁ by at least about 0.125 inch (about 0.32 cm), at least about 0.25 inch, at least about 0.5 inch, at least about 0.75 inch, at least about 1 inch, at least about 1.25 inches, at least about 1.5 inches, at least about 1.75 inches, at least about 2 inches, about 0.125 inch to about 2 inches, about 0.125 inch to about 0.25 inch, about 0.25 inch to about 0.5 inch, about 0.5 inch to about 0.75 inch, about 0.75 inch to about 1 inch, about 1 inch to about 1.25 inches, about 1.25 inches to about 1.5 inches, about 1.5 inches to about 1.75 inches, about 1.75 inches to about 2 inches, about 0.125 inch, about 0.5 inch, about 0.75 inch, about 1 inch, about 1.25 inches, about 1.5 inches, about 1.75 inches, or about 2 inches. BD₂ may be greater than OD₂ by at least about 0.125 inch (about 0.32 cm), at least about 0.25 inch, at least about 0.5 inch, at least about 0.75 inch, at least about 1 inch, at least about 1.25 inches, at least about 1.5 inches, at least about 1.75 inches, at least about 2 inches, about 0.125 inch to about 2 inches, about 0.125 inch to about 0.25 inch, about 0.25 inch to about 0.5 inch, about 0.5 inch to about 0.75 inch, about 0.75 inch to about 1 inch, about 1 inch to about 1.25 inches, about 1.25 inches to about 1.5 inches, about 1.5 inches to about 1.75 inches, about 1.75 inches to about 2 inches, about 0.125 inch, about 0.5 inch, about 0.75 inch, about 1 inch, about 1.25 inches, about 1.5 inches, about 1.75 inches, or about 2 inches.

The opening 106 is formed in and extends through the fluid impermeable barrier 102, thereby enabling fluids to enter the chamber 104 from outside of the fluid collection device 100. The opening 106 may be configured to be positioned adjacent to a female urethra, positioned adjacent to the skin of a female over the urethra, or positioned adjacent to the skin over the penis of a male having a buried penis. A fluid collection device having at least one of a greater lateral width BD₁ of the fluid impermeable barrier 102 or a greater lateral width OD₁ of the opening 106 than conventional fluid collection devices allows for easier positioning and securement of the fluid collection device 100 on thinner patients. As noted above, a substantially flat, wider fluid collection device 100 may use the inner thighs or seam between the thighs and pelvis for more secure positioning of the fluid collection device 100 against the urethra of a female user. In some embodiments, the back side of the fluid impermeable barrier 102 may include a crease or seam that enables the fluid impermeable barrier to more easily achieve a bi-fold configuration for more secure positioning of the fluid collection device 100 between the legs and against the urethra of a female user. The crease or seam may be positioned on the back side of the fluid impermeable barrier and substantially aligned with the axis 107. In another example of a man having a buried penis, a substantially flat, wider fluid collection device 102 may be placed on the skin of the male over the buried penis. The wider fluid collection device 100 covers more surface area on the male to allow the fluid collection device to collect more of the discharged urine from folds in the skin leading to the buried penis. The wider fluid collection device 100 also may be used to cover and collect fluid discharge from a wound.

With the fluid collection device 100 positioned proximate to the female urethra, positioned adjacent to the skin of a female over the urethra, or positioned adjacent to the skin over the penis of a male having a buried penis, urine may enter the interior region or chamber 104 of the fluid collection device 100 via the opening 106. Accordingly, the fluid collection device 100 is configured to receive the fluids into the chamber 104 via the opening 106. In an embodiment, the fluid impermeable barrier 102 may be configured to be attached to the individual, such as adhesively attached (*e.g.,* with a hydrogel adhesive) to the individual. For example, an adhesive can be used to secure at least a portion of the lip surrounding the opening to the individual. According to an embodiment, a suitable adhesive is a hydrogel layer, such as those disclosed in U.S. Patent Application Publication No. 2017/0189225.

The fluid impermeable barrier 102 may also temporarily store the fluids in the chamber 104. For example, the fluid impermeable barrier 102 may be formed of any suitable fluid impermeable materials, such as a fluid impermeable polymer (*e.g.,* silicone, polypropylene, polyethylene, polyethylene terephthalate, a polycarbonate, etc.), polyurethane films, thermoplastic elastomer (TPE), rubber, thermoplastic polyurethane, oil, another suitable material, or combinations thereof. As such, the fluid impermeable barrier 102 substantially prevents the fluids from exiting the portions of the chamber 104 that are spaced from the opening 106. The fluid impermeable barrier is flexible, allowing the fluid collection device 100 to bend or curve when positioned against the body of a wearer. Example fluid impermeable barriers may include, but are not limited to, a fluid impermeable barrier including at least one of Versa flex CL 2000X TPE, Dynaflex G6713 TPE, or Silpuran 6000/05 A/B silicone. In an embodiment, the fluid impermeable barrier 102 may be air permeable and fluid impermeable. In such an embodiment, the fluid impermeable barrier 102 may be formed of a hydrophobic material that defines a plurality of pores. The fluid impermeable barrier 102 may include markings thereon, such as one or more markings to aid a user in aligning the device 100 on the wearer. For example, a line on the fluid impermeable barrier 102 (*e.g.,* opposite the opening 106) may allow a healthcare professional to align the opening 106 over the urethra or penis of the wearer. In examples, the markings may include one or more of alignment guide or an orientation indicator, such as a stripe or hashes. Such markings may be positioned to align the device 100 to one or more anatomical features such as a pubic bone, etc.

The fluid collection device 100 includes a fluid permeable body 120 or layer disposed in the chamber 104. The fluid permeable body 120 extends across at least a portion (*e.g.,* all) of the opening 106. For example, the fluid permeable body 120 includes a top or first surface 111 (shown in **FIGS. 2A** and **2B**) extending across at least a portion of the opening 106 and a bottom or second surface 109 (shown in **FIGS. 2A** and **2B**) opposite to the first surface 111. At least a majority (*e.g.,* all) of the second surface 109 may interface an inner surface 103 of the fluid impermeable barrier 102. In an embodiment, the fluid permeable body 120 and at least a portion of the conduit 108 may at least substantially completely fill the chamber 104. In another example, the fluid permeable body 120 and at least a portion of the conduit may not substantially completely fill the chamber 104. In such an example, the fluid collection device 100 includes the reservoir 122 disposed in the chamber 104, described in greater detail below. The fluids that are in the chamber 104 may flow through the fluid permeable body 120 to the reservoir 122. In these and other embodiments, the fluid permeable body 120, at least a portion of the conduit 108, and the reservoir 122 may at least substantially completely fill the chamber 104. In some embodiments, the fluid permeable body 120 extends at least partially into the protrusion 127 of the fluid impermeable barrier 102. The fluid permeable body 120 also may be absent from the protrusion 127 of the fluid impermeable barrier 102.

The fluid permeable body 120 may be configured to wick any fluid away from the opening 106, thereby preventing the fluid from escaping the chamber 104. The fluid permeable body 120 also may wick the fluid generally towards an interior of the chamber 104, as discussed in more detail below. A portion of the fluid permeable body 120 may define a portion of an outer surface of the fluid collection device 100. Specifically, the portion of the fluid permeable body 120 defining the portion of the outer surface of the fluid collection device 100 may be the portion of the fluid permeable body 120 exposed by the opening 106 defined by the fluid impermeable barrier 102 that contacts the user. Moreover, the portion of the fluid permeable device defining the portion of the outer surface of the fluid collection device 100 may be free from coverage by gauze or other wicking material at the opening.

The fluid permeable body 120 includes any material that may wick the fluid. The permeable properties referred to herein may be wicking, capillary action, diffusion, or other similar properties or processes, and are referred to herein as "permeable" and/or "wicking." Such "wicking" may exclude absorption into the wicking material. The fluid permeable body 120 may include a one-way fluid movement fabric. As such, the fluid permeable body 120 may remove fluid from the area around the female urethra, thereby leaving the urethra dry. The fluid permeable body 120 may enable the fluid to flow generally towards a reservoir 122 (shown in **FIG. 3**) of void space formed within the chamber 104. For example, the fluid permeable body 120 may include a porous or fibrous material, such as hydrophilic polyolefin. In some embodiments, the fluid permeable body 120 consists of or consists essentially of a porous or fibrous material, such as hydrophilic polyolefin. Examples of polyolefin that may be used in the fluid permeable body 120 include, but are not limited to, polyethylene, polypropylene, polyisobutylene, ethylene propylene rubber, ethylene propylene diene monomer, or combinations thereof. The porous or fibrous material may be extruded into a substantially flat or planar shape to fit within the chamber 104 of the fluid impermeable barrier 102. The fluid permeable body 120 may be shaped generally complementary to the shape of the fluid impermeable barrier 102. For example, the fluid permeable body 120 may include at least a substantially flat or planar portion that extends across at least a portion of the opening 106. The fluid permeable body 120 also may additionally or alternatively include a substantially flat or planar portion that interfaces the back side of the fluid impermeable barrier 102 opposite of the opening 106. The fluid permeable body 120 may include varying densities or dimensions. Moreover, the fluid permeable body 120 may be manufactured according to various manufacturing methods, such as molding, extrusion, or sintering.

In some embodiments, the fluid permeable body 120 includes a singular and porous body. That is, during use, the fluid permeable body 120 extends from the conduit 108 to interface the fluid impermeable barrier 102 and the opening 106. A singular fluid permeable body 120 is advantageous to conventional systems, which typically require an air-laid nonwoven pad covered by a ribbed fabric compression bandage, because a singular fluid permeable body 120 reduced the number of components in the fluid collection device 100, reduced the assembly time of the fluid collection device 100, required shelf-life data for only a single component, and provided a latex-free single component. In some embodiments, at least a portion of the singular porous material of the fluid permeable body 120 extends continuously between the opening 106 and the reservoir 122 to wick any fluid from the opening 106 directly to the reservoir 122. Moreover, as the fluid impermeable barrier 102 is flexible and the fluid permeable body 120 is configured to wick fluid from the body rather than absorb fluid from the body and hold the fluid against the body, the fluid collection device 100, in some embodiments, is free from a seal or cushioning ring on the inward edge 129 defining the opening 106. In these and other embodiments, the fluid permeable body 120 includes an outer surface and a single layer or type of material between the opening 106 and the conduit 108 positioned within the fluid permeable body 120.

In other embodiments, the fluid permeable body 120 may include two or more layers of fluid permeable materials and include no (or an absence of) more than two layers of material between the opening 106 and the conduit 108 positioned within the fluid permeable body 120. For example, the fluid collection device 100 may include a fluid permeable membrane covering or wrapping around at least a portion of a fluid permeable body, with both the fluid permeable membrane and the fluid permeable body being disposed in the chamber 104. The fluid permeable membrane may cover or extend across at least a portion (*e.g.,* all) of the opening 106. The fluid permeable membrane may be configured to wick any fluid away from the opening 106, thereby preventing the fluid from escaping the chamber 104. The permeable properties referred to herein may be wicking, capillary action, diffusion, or other similar properties or processes, and are referred to herein as "permeable" and/or "wicking." In some embodiments, at least one of the fluid permeable membrane or the fluid permeable support include nylon configured to wick fluid away from the opening 106. The material of the fluid permeable membrane and the fluid permeable support also may include natural fibers. In such examples, the material may have a coating to prevent or limit absorption of fluid into the material, such as a water repellent coating. Such "wicking" may not include absorption into the wicking material. Put another way, substantially no absorption of fluid into the material may take place after the material is exposed to the fluid and removed from the fluid for a time, or substantially all or most of the fluid is absent from the wicking material and/or the fluid permeable body 120 after the material is exposed to the fluid and removed from the fluid for a time. While no absorption is desired, the term "substantially no absorption" may allow for nominal amounts of absorption of fluid into the wicking material (*e.g.,* absorbency), such as less than about 10 wt% of the dry weight of the wicking material, less than about 7 wt%, less than about 5 wt%, less than about 3 wt%, less than about 2 wt%, less than about 1 wt%, or less than about 0.5 wt% of the dry weight of the wicking material.

The fluid permeable membrane may also wick the fluid generally towards an interior of the chamber 104, as discussed in more detail below. The fluid permeable membrane may include any material that may wick the fluid. For example, the fluid permeable membrane may include fabric, such as a gauze (*e.g.,* a silk, linen, polymer based materials such as polyester, or cotton gauze), nylon, another soft fabric (*e.g.,* jersey knit fabric or the like), or another smooth fabric (*e.g.,* rayon, satin, or the like). Forming the fluid permeable membrane from gauze, soft fabric, and/or smooth fabric may reduce chaffing caused by the fluid collection device 100. Other embodiments of fluid permeable membranes, fluid permeable supports, chambers, and their shapes and configurations are disclosed in U.S. Patent Application No. 15/612,325 filed on June 2, 2017; U.S. Patent Application No. 15/260,103 filed on September 8, 2016; U.S. Patent Application No. 15/611,587 filed on June 1, 2017; PCT Patent Application No. PCT/US19/29608, filed on April 29, 2019. In many embodiments, the fluid permeable body 120 includes a fluid permeable support including a porous nylon structure (*e.g.,* spun nylon fibers) and a fluid permeable membrane including gauze about or over the porous nylon structure.

**FIG. 2A** is a cross-sectional view of the fluid collection device 100 taken along line 2-2 of **FIG. 1****,** and **FIG. 2B** is a cross-sectional view of the fluid collection device 100 taken along line 2-2 of **FIG. 1** and with the conduit 108 removed. The fluid permeable body 120 also includes a slot sized to at least partially receive the conduit 108 therein. The slot extends longitudinally along the fluid permeable body 120 and may extend from and the second surface 109 to a suitable depth within the fluid permeable body 120. The slot enables the conduit 108 to be positioned within the fluid permeable body 120 with pushing the conduit 108 through the longitudinal length of the fluid permeable body 120.

The slot may be configured as an L-shaped (not shown) or T-shaped slot having a first portion 204 generally planar and substantially parallel to the first surface 111 and the second surface 109 of the fluid permeable body 120, and a second portion 206 substantially perpendicular or otherwise angled relative to the first surface 111 and the second surface 109 of the fluid permeable body 120. The second portion 206 of the slot may extend from the second surface 109 to the first portion 204 of the slot. The second portion 206 of the slot enables access to the first portion 204 of the slot through which the conduit 108 may be inserted. The first portion 204 of the slot may be positioned about halfway or less than from the second surface 109 to the first surface 111 of the fluid permeable body 120. The first portion 204 and the second portion 206 of the slot may include cuts or divisions in the fluid permeable body 120, thereby allowing movement through and positioning of the conduit 108 within the slot of the fluid permeable body 120. At least one of the first portion 204 or the second portion 206 of the slot may be configured to allow adjacent portions of the fluid permeable body 120 divided by the first portion 204 or the second portion 206 to interface one other at the respective first portion 204 or the second portion 206. In some embodiments, at least one of the first portion 204 or the second portion 206 of the slot may be configured to provide a gap or space between portions of the fluid permeable body 120 divided by the first portion 204 or the second portion 206 of the slot. In some embodiments, the slot may be configured as a I-shaped slot (not shown) that is substantially perpendicular or otherwise angled relative to the first surface 111 and the second surface 109 of the fluid permeable body 120. The slot may extend from the second surface 109 to a depth within the fluid permeable body. For example, the slot may extend no more than halfway into the fluid permeable body 120.

In some embodiments, the slot includes a bore or channel sized complementary to the conduit 108. In embodiments having a bore or channel sized complementary to the conduit 108, the slot may include a second portion 206 leading from the second surface 109 of the fluid permeable body 120 to the bore or channel. Alternatively, the bore or channel may extend longitudinally through the fluid permeable body 120 without a slot leading from the second surface 109 to the bore or channel.

The fluid permeable body 120 also may include one or more covers 202 at least partially covering the slot or covering at least a portion of the slot. For example, as shown in **FIG. 2B****,** the T-shaped slot forms two covers 202 covering the first portion 204 of the slot. The two covers 202 may interface one another at the second portion 206 of the slot, thereby substantially enveloping the conduit 108 within the fluid permeable body 120. For example, the conduit 108 may be inserted into the slot by moving the conduit 108 through the second portion 206 of the slot between the two covers 202 until the conduit 108 reaches the first portion 204 of the slot.

**FIG. 3** is a cross-sectional view of the fluid collection device 100 taken along line 3-3 of **FIG. 1****.** The fluid collection device 100 also includes the conduit 108 that is at least partially disposed in the chamber 104. The conduit 108 (*e.g*., a tube) includes an inlet 110 at the protrusion 127 of the fluid impermeable barrier 102 and an outlet 112 at the protrusion 125 of the fluid impermeable barrier 102 positioned downstream from the inlet 110. The conduit 108 provides fluid communication between an interior region of the chamber 104 and a fluid storage container (not shown) or a portable vacuum source (not shown). For example, the conduit 108 may directly or indirectly fluidly couple the interior region of the chamber 104 and/or the reservoir 122 with the fluid storage container or the portable vacuum source. With the conduit 108 positioned within the fluid permeable body 120, when the fluid collection device 100 is secured to a user with any of a number of securing devices, fluids received in the chamber 104 may be removed through the conduit 108.

In the illustrated embodiment, the conduit 108 is at least partially disposed in the chamber 104 and interfaces at least a portion of the slot of the fluid permeable body 120. For example, the conduit 108 may extend into the fluid impermeable barrier 102 from the protrusion 125 (*e.g*., proximate to the outlet 112) and may extend through the slot in the fluid permeable body 120 to the protrusion 127 (*e.g.,* opposite the protrusion 125) to a point proximate to the reservoir 122 such that the inlet 110 is in fluid communication with the reservoir 122. For example, in the illustrated embodiment, the inlet 110 is positioned in the reservoir 122. However, in other embodiments, the inlet 110 may be positioned flush with or behind an end of the fluid permeable body 120 that partially defines the reservoir 122. The fluid collected in the fluid collection device 100 may be removed from the interior region of the chamber 104 via the conduit 108. The conduit 108 may include a flexible material such as plastic tubing (*e.g*., medical tubing). Such plastic tubing may include a thermoplastic elastomer, polyvinyl chloride, ethylene vinyl acetate, polytetrafluoroethylene, etc., tubing. In some embodiments, the conduit 108 may include silicone or latex.

The fluid impermeable barrier 102 may store fluids in the reservoir 122 therein. The reservoir 122 is an unoccupied portion of the chamber 104 and is void of other material. In some embodiments, the reservoir 122 is defined at least partially by the fluid permeable body 120 and the fluid impermeable barrier 102. The reservoir 122 may be disposed in any portion of the interior region of the chamber 104. For example, the fluid reservoir 122 may be positioned at least partially or entirely in the protrusion 127 of the chamber 104. In the illustrated embodiment, the reservoir 122 is defined by at least a portion of an end 123 of the fluid permeable body 120 and the protrusion 127 of the fluid impermeable barrier 102. In an embodiment, the reservoir 122 may be located at the portion of the chamber 104 that is closest to the inlet 110 (*e.g*., the second end region). However, the reservoir 122 may be located at different locations in the chamber 104. In some embodiments, the conduit 108 may extend through the fluid impermeable barrier 102 to the reservoir 122 without extending through the fluid permeable body 120. Accordingly, in these and other embodiments, the fluid permeable body 120 may be free from the bore (*e.g*., the bore may be absent from the fluid permeable body 120). In another embodiment, the fluid collection device 100 may include multiple reservoirs, such as a first reservoir that is located at the portion of the chamber of the chamber 104 that is closest to the inlet 110 (*e.g.*, second end region) and a second reservoir that is located at the portion of the chamber 104 that is closest to the outlet 112 (*e.g*., first end region). In another example, the fluid permeable body 120 is spaced from at least a portion of the conduit 108 and the reservoir 122 may be the space between the fluid permeable body 120 and the conduit 108.

Other embodiments of reservoirs, fluid impermeable barriers, fluid permeable membranes, fluid permeable bodies, chambers, and their materials and configurations may are disclosed in U.S. Patent Application No. 15/612,325 filed on June 2, 2017; U.S. Patent Application No. 15/260,103 filed on September 8, 2016; and U.S. Patent Application No. 15/611,587 filed on June 1, 2017.

The fluid impermeable barrier 102, the fluid permeable body 120 may be configured to have the conduit 108 at least partially disposed in the chamber 104. For example, the fluid permeable body 120 may be configured to form a space that accommodates the conduit 108, such as the slot. In another example, the fluid impermeable barrier 102 may define an aperture 124 sized to receive the conduit 108 (*e.g*., at least one tube). The at least one conduit 108 may be disposed in the chamber 104 via the aperture 124. The apertures 124 may be configured to form an at least substantially fluid tight seal against the conduit 108 or the at least one tube thereby substantially preventing the fluids from escaping the chamber 104.

In some embodiments, the conduit 108 may extend through the fluid permeable body 120 and at least partially into the reservoir 122 and/or the protrusion 127. In some embodiments, the conduit 108 may extend through the fluid permeable body 120 and terminate at or before the body 123 of the fluid permeable body 120 such that the conduit 108 does not extend into the reservoir 122 (or the reservoir 122 is absent of the conduit 108). For example, an end (such as the inlet 110) of the conduit 108 may be generally flush or coplanar with the end 123 of the fluid permeable body 120. In other embodiments, the end of the conduit 108 may be recessed from the end 123 of the fluid permeable body 120. The end of the conduit 108 also may be selectively moveable between partially extending into the reservoir 122 and recessed from or flush with the end 123 of the fluid permeable body 120.

When secured to the fluid collection device 100, the conduit 108 is configured to provide fluid communication with and at least partially extend between one or more of a fluid storage containers (not shown) and a portable vacuum source (not shown). For example, the conduit 108 may be configured to be fluidly coupled to and at least partially extend between one or more of the fluid storage containers and the portable vacuum source. In an embodiment, the conduit 108 is configured to be directly connected to the portable vacuum source (not shown). In such an example, the conduit 108 may extend from the fluid impermeable barrier 102 by at least one foot, at least two feet, at least three feet, or at least six feet. In another example, the conduit 108 is configured to be indirectly connected to at least one of the fluid storage container (not shown) or the portable vacuum source (not shown). In some examples, the conduit may be frosted or opaque (*e.g.,* black) to obscure visibility of the fluids therein. In some embodiments, the conduit is secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063.

The inlet 110 and the outlet 112 are configured to provide fluid communication (*e.g.,* directly or indirectly) between the portable vacuum source (not shown) and the chamber 104 (*e.g.,* the reservoir 122). For example, the inlet 110 and the outlet 112 of the conduit 108 may be configured to directly or indirectly fluidly couple the portable vacuum source to the reservoir 122. In an embodiment, the inlet 110 and/or the outlet 112 may form a male connector. In another example, the inlet 110 and/or the outlet 112 may form a female connector. In an embodiment, the inlet 110 and/or the outlet 112 may include ribs that are configured to facilitate secure couplings. In an embodiment, the inlet 110 and/or the outlet 112 may form a tapered shape. In an embodiment, the inlet 110 and/or the outlet 112 may include a rigid or flexible material.

Locating the inlet 110 at or near a gravimetrically low point of the chamber 104 enables the conduit to receive more of the fluids than if inlet 110 was located elsewhere and reduce the likelihood of pooling (*e.g.,* pooling of the fluids may cause microbe growth and foul odors). For instance, the fluids in the fluid permeable body 120 may flow in any direction due to capillary forces. However, the fluids may exhibit a preference to flow in the direction of gravity, especially when at least a portion of the fluid permeable body 120 is saturated with the fluids.

As the portable vacuum source applies a vacuum/suction in the conduit 108, the fluid(s) in the chamber 104 (*e.g.,* such as in the reservoir 122 positioned at the first end region 125, the second end region 127, or other intermediary positions within the chamber 104) may be drawn into the inlet 110 and out of the fluid collection device 100 via the conduit 108.

In an embodiment, the conduit 108 is configured to be at least insertable into the chamber 104. In such an embodiment, the conduit 108 may include one or more markers on an exterior thereof that are configured to facilitate insertion of the conduit 108 into the chamber 104. For example, the conduit 108 may include one or more markings thereon that are configured to prevent over or under insertion of the conduit 108, such as when the conduit 108 defines an inlet 110 that is configured to be disposed in or adjacent to the reservoir 122. In another embodiment, the conduit 108 may include one or more markings thereon that are configured to facilitate correct rotation of the conduit 108 relative to the chamber 104. In an embodiment, the one or more markings may include a line, a dot, a sticker, or any other suitable marking. In examples, the conduit 108 may extend into the fluid impermeable barrier 102 from the first end region (*e.g.,* proximate to the outlet 112) and may extend to the second end region (*e.g.,* opposite the first end region) to a point proximate to the reservoir 122 such that the inlet 110 is in fluid communication with the reservoir 122. In some embodiments (not shown), the conduit 108 may enter the second end region and the inlet 110 may be disposed in the second end region (*e.g.,* in the reservoir 122). The fluid collected in the fluid collection device 100 may be removed from the interior region of the chamber 104 via the conduit 108. The conduit 108 may include a flexible material such as plastic tubing (*e.g.,* medical tubing) as disclosed herein. In some examples, the conduit 108 may include one or more portions that are resilient, such as to by having one or more of a diameter or wall thickness that allows the conduit to be flexible.

In an embodiment, one or more components of the fluid collection device 100 may include an antimicrobial material, such as an antibacterial material where the fluid collection device may contact the wearer or the bodily fluid of the wearer. The antimicrobial material may include an antimicrobial coating, such as a nitrofurazone or silver coating. The antimicrobial material may inhibit microbial growth, such as microbial growth due to pooling or stagnation of the fluids. In an embodiment, one or more components of the fluid collection device 100 (*e.g.,* impermeable barrier 102, conduit 108, etc.) may include an odor blocking or absorbing material such as a cyclodextrine containing material or a TPE polymer.

In any of the embodiments disclosed herein the conduits 108 may include or be operably coupled to a flow meter (not shown) to measure the flow of fluids therein, one or more securement devices (*e.g.,* a StatLock securement device, not shown) or fittings to secure the conduit 108 to one or more components of the systems or devices disclosed herein (*e.g.,* portable vacuum source or fluid storage container), or one or more valves to control the flow of fluids in the systems and devices herein.

In an embodiment, at least one of portion of the conduit 108 of the fluid collection devices or systems herein may be formed of an at least partially opaque material which may obscure the fluids that are present therein. For example, a first section of the conduit 108 disclosed herein may be formed of an opaque material or translucent material while a second section may be formed of a transparent material or translucent material. In some embodiments, the first section may include transparent or translucent material. Unlike the opaque or nearly opaque material, the translucent material allows a user of the devices and systems herein to visually identify fluids or issues that are inhibiting the flow of fluids within the conduit 108.

In any of the examples, systems or devices disclosed herein, the system of fluid collection device may include moisture sensors (not shown) disposed inside of the chamber of the fluid collection device. In such examples, the moisture sensor may be operably coupled to a controller or directly to the portable vacuum source, and may provide electrical signals indicating that moisture is or is not detected in one or more portions of the chamber. The moisture sensor(s) may provide an indication that moisture is present, and responsive thereto, the controller or portable vacuum device may direct the initiation of suction to the chamber to remove the fluid therefrom. Suitable moisture sensors may include capacitance sensors, volumetric sensors, potential sensors, resistance sensors, frequency domain reflectometry sensors, time domain reflectometry sensors, or any other suitable moisture sensor. In practice, the moisture sensors may detect moisture in the chamber and may provide a signal to the controller or portable vacuum source to activate the portable suction device.

**FIG. 4** is a cross-section view of the fluid impermeable barrier 102 of the fluid collection device 100 taken along line 3-3 thereof, with the fluid permeable body 120 and the conduit 108 removed. **FIG. 4** illustrates the chamber 104 defined at least partially by the inner surface 103 of the fluid impermeable barrier 102, as well as the aperture 124 defined by the protrusion 125. In some embodiments, the fluid impermeable barrier 102 does not include the protrusion 127 or the protrusion 127 is absent, and the aperture 124 may be defined by the first lateral side 140a or another portion of the fluid impermeable barrier 102.

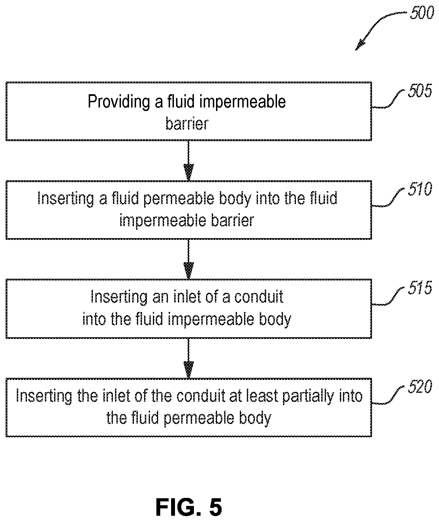

**FIG. 5** is a flow diagram of a method 500 of assembling the fluid collection devices and/or fluid collection systems disclosed herein. The method 500 may include act 505, which recites providing a fluid impermeable barrier. The fluid impermeable barrier may include any fluid impermeable barrier described herein, such as the fluid impermeable barrier 102. The fluid impermeable barrier at least partially defines a chamber and also an opening extending therethrough. The opening is configured to be positioned adjacent to a female urethra, positioned adjacent to the skin of a female over the urethra, positioned adjacent to the skin over the penis of a male having a buried penis, or positioned over a wound.

The method may include act 510, which recites inserting a fluid permeable body into the chamber of the fluid impermeable barrier. The fluid permeable body may include any fluid permeable body described herein, such as the fluid permeable body 120. When the fluid permeable body is inserted into the chamber of the fluid impermeable barrier, the fluid permeable body interfaces at least a portion of the fluid impermeable barrier and covers at least a portion of the opening. The fluid permeable body is configured to wick any fluid away from the opening. In some embodiments, act 510 may include inserting or positioning the fluid permeable body in the chamber of the fluid impermeable barrier such that a reservoir is defined within the chamber by a protrusion of the fluid permeable body distal to the aperture. In some embodiments, act 510 may include inserting or positioning the fluid permeable body in the chamber defined by the fluid impermeable barrier with a conduit in fluid communication with the reservoir positioned in and at least partially defined by the protrusion of the fluid impermeable barrier that is distal to the aperture. In some embodiments, act 510 may include inserting or positioning the fluid permeable body in the chamber of the fluid impermeable barrier such that the fluid permeable body, the conduit, and the reservoir fill substantially all of the chamber.

The method may include act 515, which recites inserting an inlet of a conduit into the fluid impermeable body. The conduit may be inserted into the fluid impermeable body through an aperture defined by the fluid impermeable barrier at a protrusion of the fluid impermeable barrier.

The method may include an act 520, which recites inserting at least a portion of conduit (such as the inlet of the conduit) at least partially into the slot extending longitudinally through of the fluid permeable body. The slot extends at least partially through the fluid permeable body and is defined by the fluid permeable body. The conduit interfaces at least a portion of the fluid permeable body. In some embodiments, inserting the conduit into the slot of the fluid permeable body includes inserting the conduit into a T-shaped slot extending longitudinally through the fluid permeable body such that one or more covers of the fluid permeable body at least partially cover the slot and envelop the conduit within the fluid permeable body. The fluid permeable body may be pinched to open the T-shaped slot between two covers of the fluid permeable body to allow for insertion of the conduit.

Acts 505, 510, 515, and 520 of the method 500 are for illustrative purposes. For example, acts 505, 510, 515, and 520 of the method 500 may be performed in different orders, split into multiple acts, modified, supplemented, or combined. For example, in some embodiments, act 510 precedes acts 515 and 520, and in other embodiments, acts 515 and 520 precede act 510. In an embodiment, one or more of acts 505, 510, 515, and 520 of the method 500 may be omitted from the method 500. Any of acts 505, 510, 515, and 520 may include using any of the fluid collection devices or systems disclosed herein.

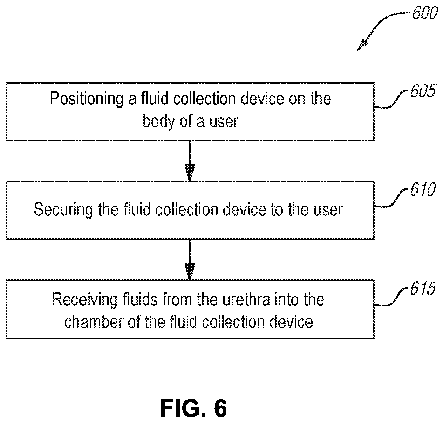

**FIG. 6** is a flow diagram of a non-claimed method 600 for collecting fluids. In particular examples, the method 600 includes a method for collecting fluids from a user having a buried penis. The method 600 includes an act 605 of positioning a fluid collection device on the body of a user. Act 605 may include positioning an opening of the fluid collection device over the buried penis of the user, with the opening being defined by a fluid impermeable barrier and including a longitudinal length of at least about 4 cm and a lateral width that is substantially equal to or greater than the longitudinal length. In some embodiments, act 605 may include positioning the opening of the fluid collection device over or adjacent to a female urethra of a user, positioning the opening of the fluid collection device over or adjacent to the skin of a female over the urethra, , or positioning the opening of the fluid collection device over a wound. The fluid impermeable barrier may include any fluid impermeable barrier described herein, such as the fluid impermeable barrier 102. In some embodiments, positioning the opening of the fluid collection device over the buried penis of the user includes positioning the fluid permeable body of the fluid collection device adjacent to the skin over the buried penis. The fluid permeable body is disposed within a chamber of a fluid impermeable barrier of the fluid collection device and exposed to the body of the user through an opening in the fluid collection device defined by the fluid impermeable barrier.

The method 600 also includes an act 610 of securing the fluid collection device to the user. The method 600 also includes an act 615 of receiving fluids from the user into the chamber of the fluid collection device. Act 615 may include receiving fluids in a reservoir positioned in and at least partially defined by a protrusion of the fluid impermeable barrier that is distal. In some embodiments, the method 600 includes an act of applying suction effective to suction the fluids from the chamber via a conduit disposed therein.

Acts 605, 610, and 615 of the method 600 are for illustrative purposes. For example, the acts 605, 610, and 615 of the method 600 may be performed in different orders, split into multiple acts, modified, supplemented, or combined. In an embodiment, one or more of the acts 605, 610, and 615 of the method 600 may be omitted from the method 600. Any of the acts 605, 610, and 615 may include using any of the fluid collection devices or systems disclosed herein.

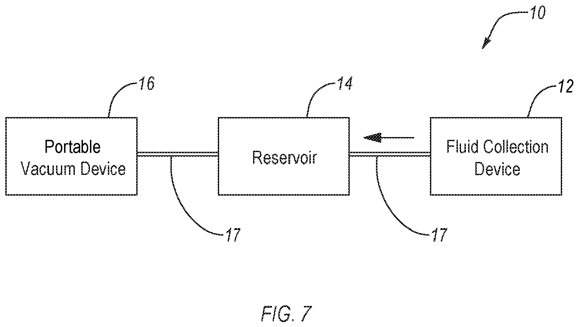

**FIG. 7** is a block diagram of a system 10 for fluid collection. The system 10 includes a fluid collection device 12, a fluid storage container 14, and a portable vacuum source 16. The fluid collection device 12 may include any of the fluid collection devices described herein, such as the fluid collection device 100. The fluid collection device 12, the fluid storage container 14, and the portable vacuum source 16 may be fluidly coupled to each other via one or more conduits 17. The conduit 17 may include any of the conduits described herein, such as the conduit 108. The fluid collection device 12 may be operably coupled to one or more of the fluid storage container 14 or the portable vacuum source via the conduit 17. Fluid (*e.g*., urine or other bodily fluids) collected in the fluid collection device 12 may be removed from the fluid collection device 12 via the conduit 17, which protrudes into an interior region of the fluid collection device 12. For example, a first open end of the conduit 17 may extend into the fluid collection device 12 to a reservoir therein. The second open end of the conduit 17 may extend into the fluid collection device 12 or the portable vacuum source 16. The suction force may be introduced into the interior region of the fluid collection device 12 via the first open end of the conduit 17 responsive to a suction (*e.g*., vacuum) force applied at the second end of the conduit 17. The suction force may be applied to the second open end of the conduit 17 by the portable vacuum source 16 either directly or indirectly.

The suction force may be applied indirectly via the fluid storage container 14. For example, the second open end of the conduit 17 may be disposed within the fluid storage container 14 and an additional conduit 17 may extend from the fluid storage container 14 to the portable vacuum source 16. Accordingly, the portable vacuum source 16 may apply suction to the fluid collection device 12 via the fluid storage container 14. The suction force may be applied directly via the fluid storage container 14. For example, the second open end of the conduit 17 may be disposed within the portable vacuum source 16. An additional conduit 17 may extend from the portable vacuum source 16 to a point outside of the fluid collection device 12, such as to the fluid storage container 14. In such examples, the portable vacuum source 16 may be disposed between the fluid collection device 12 and the fluid storage container 14.

The fluid collection device 12 may be shaped and sized to be positioned adjacent to a female urethra or on the skin over a buried penis of a male. The fluid collection member of the fluid collection device 12 may include a fluid impermeable barrier at least partially defining a chamber (*e.g.,* interior region of the fluid collection device member) of the fluid collection device 12. As described in more detail above, the fluid collection device 12 may include a wider opening than conventional fluid collection devices. The fluid impermeable barrier also defines an opening extending therethrough from the external environment. The opening may be positioned on the fluid collection member to be aligned adjacent to a female urethra or over a buried penis of a male. The fluid collection member of the fluid collection device 12 may include a fluid permeable body disposed within the fluid impermeable barrier. The fluid permeably body may include a fluid permeable membrane and fluid permeable support disposed within the fluid permeable membrane. The conduit 17 may extend into the fluid collection device 12 at a first end region, through one or more of the fluid impermeable barrier, fluid permeable membrane, or the fluid permeable support to a second end region of the fluid collection member of the fluid collection device 12. Example fluid collection devices for use with the systems and methods herein are described in more detail below.

In some embodiments, the fluid storage container 14 may include a bag (*e.g.,* drainage bag), a bottle or cup (*e.g.,* collection jar), or any other enclosed container for storing bodily fluids such as urine. In examples, the conduit 17 may extend from the fluid collection device 12 and attach to the fluid storage container 14 at a first point therein. An additional conduit 17 may attach to the fluid storage container 14 at a second point thereon and may extend and attach to the portable vacuum source 16. For example, the fluid storage container 14 may include a container fluidly coupled to a first conduit section that is also fluidly coupled to the fluid collection member of the fluid collection device 12. The container may be fluidly coupled to a second section of the conduit 17 that is also fluidly coupled to a portable vacuum source. In such examples, the portable vacuum source 16 may provide a vacuum/suction through the container to the fluid collection member to provide suction in the chamber of the fluid collection member. Accordingly, a vacuum (*e.g.,* suction) may be drawn through fluid collection device 12 via the fluid storage container 14. As the fluid is drained from the chamber, the fluid may travel through the first section of conduit to the fluid storage container where it may be retained. Fluid, such as urine, may be drained from the fluid collection device 12 using the portable vacuum source 16.

In some embodiments, the portable vacuum source 16 may be disposed in or on the fluid collection device 12. In such examples, the conduit 17 may extend from the fluid collection device and attach to the portable vacuum source 16 at a first point therein. An additional conduit 17 may attach to the portable vacuum source 16 at a second point thereon and may extend out of the fluid collection device 12, and may attach to the fluid storage container 14. Accordingly, a vacuum (*e.g.,* suction) may be drawn through fluid collection device 12 via the fluid storage container 14.

The portable vacuum source 16 may include one or more of a manual vacuum pump, and electric vacuum pump, a diaphragm pump, a centrifugal pump, a displacement pump, a magnetically driven pump, a peristaltic pump, or any pump configured to produce a vacuum. The portable vacuum source 16 may provide a vacuum or suction to remove fluid from the fluid collection member of the fluid collection device 12. In some embodiments, the portable vacuum source 16 may be powered by one or more of a power cord (*e.g.,* connected to a power socket), one or more batteries, or even manual power (*e.g.,* a hand operated vacuum pump). In examples, the portable vacuum source 16 may be sized and shaped to fit outside of, on, or within the fluid collection device 12. For example, the portable vacuum source 16 may include one or more miniaturized pumps or one or more micro pumps. The portable vacuum sources 16 disclosed herein may include one or more of a switch, a button, a plug, a remote, or any other device suitable to activate the portable vacuum source 16. It should be understood that the portable vacuum sources 16 disclosed herein may provide a portable means of providing a suction or vacuum that allows use of the devices and systems herein outside of hospital or care facility environments where vacuum lines are plumbed into patient rooms or large (*e.g.,* larger or heavier than a patient may readily carry) vacuum sources are located. For example, a portable vacuum source may be small and light enough to be carried by a user (*e.g.,* patient) or aid (*e.g.,* nurse) during transportation of the user.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A substantially flat urine collection device (100) to collect urine discharged during urination, comprising:
a fluid impermeable barrier (102) having a first lateral side (140a) defining an aperture (124) configured to receive a conduit therethrough and a second lateral side (140b) distal to the first lateral side, a protrusion (127) of the fluid impermeable barrier which extends from the second lateral side, the fluid impermeable barrier also defining a chamber (104) and an opening (106), the opening including a longitudinal length (OD2) of at least about 4 cm and a lateral width (OD1); and
a fluid permeable body (120) positioned at least partially within the chamber such that a portion of the fluid permeable body extends across at least a portion of the opening and to at least partially define, with the fluid impermeable barrier, a reservoir (122) that is positioned in the protrusion (127), the fluid permeable body being configured to wick urine away from the opening to the reservoir; and wherein:
i) the lateral width (OD1) of the opening is substantially equal to or greater than the longitudinal length (OD2); and
ii) the aperture and the protrusion are positioned on an axis (107) that is substantially centered on the lateral width of the opening
the device further comprising a conduit (108) including an inlet and an outlet, wherein at least a portion of the conduit extends through the aperture and the fluid permeable body to position the inlet at the protrusion in fluid communication with the reservoir.

2. The fluid collection device of claim 1, wherein the lateral width is from 5 cm to 7.5 cm.

3. The fluid collection device of either of the preceding claims, wherein the lateral width is at least 1.5 times greater than the longitudinal length.

4. The fluid collection device of any of the preceding claims, wherein:
the fluid impermeable barrier includes two longitudinal sides (130) extending between the first lateral side and the second lateral side; and
the opening is substantially rectangular.

5. The fluid collection device of any of the preceding claims, wherein the fluid permeable body (120) is absent from the protrusion (127).

6. The fluid collection device of any one of the preceding claims, wherein the fluid permeable body includes two or fewer layers of material positioned between the conduit and the opening.

7. The fluid collection device of any one of the preceding claims, wherein the fluid permeable body includes a slot (204, 206) and the conduit is positioned at least partially within the slot.

8. The fluid collection device of claim 7, wherein the fluid permeable body includes one or more covers covering at least a portion of the slot when the conduit is at least partially positioned within the slot.

9. Device as claimed in claim 7 wherein the slot is I-shaped and extends from the second surface of the fluid permeable body to a depth within the fluid permeable body.

10. Device as claimed in claim 7 wherein the slot is L-shaped.

11. Device as claimed in claim 7 wherein the slot is T-shaped with a first portion (204) substantially parallel to the first surface and second surface of the fluid permeable body and a second portion (206) that extends from the second surface to enable the conduit to access the first portion of the slot.

12. Device as claimed in any of claims 7 to 11 in which the slot includes a bore or channel complementary to the conduit.

13. Device as claimed in any one of the preceding claims and including a crease or seam that enables the fluid impermeable barrier to more easily achieve a bi-fold configuration for more secure positioning of the fluid collection device between the legs and against the urethra of a female user, the crease or seam being aligned with the said axis.

14. Device as claimed in any one of the preceding claims and including markings on the barrier (102) to aid a healthcare professional to align the opening over the urethra or penis of a wearer of the device.

15. Device as claimed in any one of the preceding claims, in a system that includes a urine storage container.

## Patentansprüche

1. Im Wesentlichen flache Urinsammelvorrichtung (100) zum Sammeln von beim Urinieren ausgeschiedenem Urin, umfassend:
eine fluidundurchlässige Barriere (102), die eine erste laterale Seite (140a), die eine Mündung (124) definiert, die konfiguriert ist zum Aufnehmen einer Leitung dadurch, und eine zweite laterale Seite (140b), die distal zur ersten lateralen Seite ist, einen Vorsprung (127) der fluidundurchlässigen Barriere aufweist, der sich von der zweiten lateralen Seite erstreckt, wobei die fluidundurchlässige Barriere außerdem eine Kammer (104) und eine Öffnung (106) definiert, wobei die Öffnung eine Längslänge (OD2) von mindestens etwa 4 cm und eine seitliche Breite (OD1) einschließt; und
einen fluiddurchlässigen Körper (120), der zumindest teilweise innerhalb der Kammer angeordnet ist, so dass sich ein Abschnitt des fluiddurchlässigen Körpers über zumindest einen Abschnitt der Öffnung erstreckt und mit der fluidundurchlässigen Barriere zumindest teilweise ein Reservoir (122) definiert, das im Vorsprung (127) angeordnet ist, wobei der fluiddurchlässige Körper konfiguriert ist zum Abtransportieren von Urin aus der Öffnung zum Reservoir; und wobei:
i) die seitliche Breite (OD1) der Öffnung ist im Wesentlichen gleich oder größer als die Längslänge (OD2) ist; und
ii) die Mündung und der Vorsprung auf einer Achse (107) angeordnet sind, die im Wesentlichen auf der seitlichen Breite der Öffnung zentriert ist,
wobei die Vorrichtung weiter eine Leitung (108) umfasst, die einen Einlass und einen Auslass einschließt, wobei sich mindestens ein Abschnitt der Leitung durch die Mündung und den fluiddurchlässigen Körper erstreckt, um den Einlass am Vorsprung in Fluidverbindung mit dem Reservoir anzuordnen.

2. Fluidsammelvorrichtung nach Anspruch 1, wobei die seitliche Breite 5 cm bis 7,5 cm beträgt.

3. Fluidsammelvorrichtung nach einem der vorstehenden Ansprüche, wobei die seitliche Breite mindestens 1,5-mal größer ist als die Längslänge.

4. Fluidsammelvorrichtung nach einem der vorstehenden Ansprüche, wobei:
die fluidundurchlässige Barriere zwei Längsseiten (130) einschließt, die sich zwischen der ersten lateralen Seite und der zweiten lateralen Seite erstrecken; und
die Öffnung im Wesentlichen rechteckig ist.

5. Fluidsammelvorrichtung nach einem der vorstehenden Ansprüche, wobei der fluiddurchlässige Körper (120) am Vorsprung (127) fehlt.

6. Fluidsammelvorrichtung nach einem der vorstehenden Ansprüche, wobei der fluiddurchlässige Körper zwei oder weniger Materialschichten einschließt, die zwischen der Leitung und der Öffnung angeordnet sind.

7. Fluidsammelvorrichtung nach einem der vorstehenden Ansprüche, wobei der fluiddurchlässige Körper einen Schlitz (204, 206) einschließt und die Leitung zumindest teilweise innerhalb des Schlitzes angeordnet ist.

8. Fluidsammelvorrichtung nach Anspruch 7, wobei der fluiddurchlässige Körper eine oder mehrere Abdeckungen einschließt, die zumindest einen Abschnitt des Schlitzes abdecken, wenn die Leitung zumindest teilweise innerhalb des Schlitzes angeordnet ist.

9. Vorrichtung nach Anspruch 7, wobei der Schlitz I-förmig ist und sich von der zweiten Oberfläche des fluiddurchlässigen Körpers bis zu einer Tiefe innerhalb des fluiddurchlässigen Körpers erstreckt.

10. Vorrichtung nach Anspruch 7, wobei der Schlitz L-förmig ist.

11. Vorrichtung nach Anspruch 7, wobei der Schlitz T-förmig ist, wobei ein erster Abschnitt (204) im Wesentlichen parallel zur ersten Oberfläche und zweiten Oberfläche des fluiddurchlässigen Körpers ist, und ein zweiter Abschnitt (206) sich von der zweiten Oberfläche erstreckt, um es der Leitung zu ermöglichen, auf den ersten Abschnitt des Schlitzes zuzugreifen.

12. Vorrichtung nach einem der Ansprüche 7 bis 11, bei der der Schlitz eine Bohrung oder einen Kanal einschließt, die/der komplementär zur Leitung ist.

13. Vorrichtung nach einem der vorstehenden Ansprüche und einschließlich einer Falte oder Naht, die es der fluidundurchlässigen Barriere ermöglicht, leichter eine zweifache Konfiguration zu erreichen, um eine sicherere Positionierung der Fluidsammelvorrichtung zwischen den Beinen und gegen die Harnröhre einer weiblichen Benutzerin zu ermöglichen, wobei die Falte oder Naht mit der Achse ausgerichtet ist.

14. Vorrichtung nach einem der vorstehenden Ansprüche und einschließlich Markierungen auf der Barriere (102), um einem medizinischen Fachpersonal dabei zu helfen, die Öffnung über der Harnröhre oder dem Penis eines Trägers der Vorrichtung auszurichten.

15. Vorrichtung nach einem der vorstehenden Ansprüche, in einem System, das einen Urinspeicherbehälter einschließt.

## Revendications

1. Dispositif (100) de collecte d'urine sensiblement plat pour collecter l'urine évacuée pendant la miction, comprenant :
une barrière (102) imperméable au fluide présentant un premier côté (140a) latéral définissant un orifice (124) configuré pour recevoir un conduit à travers celui-ci et un deuxième côté (140b) latéral distal par rapport au premier côté latéral, une partie saillante (127) de la barrière imperméable au fluide qui s'étend à partir du deuxième côté latéral, la barrière imperméable au fluide définissant également une chambre (104) et une ouverture (106), l'ouverture comportant une longueur (OD2) longitudinale d'au moins environ 4 cm et une largeur (OD1) latérale ; et
un corps (120) perméable au fluide positionné au moins en partie dans la chambre de telle sorte qu'une partie du corps perméable au fluide s'étend en travers d'au moins une partie de l'ouverture et pour définir au moins en partie, avec la barrière imperméable au fluide, un réservoir (122) qui est positionné dans la partie saillante (127), le corps perméable au fluide étant configuré pour évacuer l'urine à partir de l'ouverture vers le réservoir ; et dans lequel :
i) la largeur (OD1) latérale de l'ouverture est sensiblement supérieure ou égale à la longueur (OD2) longitudinale ; et
ii) l'orifice et la partie saillante sont positionnés sur un axe (107) qui est sensiblement centré sur la largeur latérale de l'ouverture
le dispositif comprenant en outre un conduit (108) comportant une entrée et une sortie, dans lequel au moins une partie du conduit s'étend à travers l'orifice et le corps perméable au fluide pour positionner l'entrée au niveau de la partie saillante en communication fluidique avec le réservoir.

2. Dispositif de collecte de fluide selon la revendication 1, dans lequel la largeur latérale est de 5 cm à 7,5 cm.

3. Dispositif de collecte de fluide selon l'une ou l'autre des revendications précédentes, dans lequel la largeur latérale est au moins 1,5 fois supérieure à la longueur longitudinale.

4. Dispositif de collecte de fluide selon l'une quelconque des revendications précédentes, dans lequel :
la barrière imperméable au fluide comporte deux côtés (130) longitudinaux s'étendant entre le premier côté latéral et le deuxième côté latéral ; et
l'ouverture est sensiblement rectangulaire.

5. Dispositif de collecte de fluide selon l'une quelconque des revendications précédentes, dans lequel le corps (120) perméable au fluide est absent de la partie saillante (127).

6. Dispositif de collecte de fluide selon l'une quelconque des revendications précédentes, dans lequel le corps perméable au fluide comporte deux couches de matériau ou moins positionnées entre le conduit et l'ouverture.

7. Dispositif de collecte de fluide selon l'une quelconque des revendications précédentes, dans lequel le corps perméable au fluide comporte une fente (204, 206) et le conduit est positionné au moins en partie dans la fente.

8. Dispositif de collecte de fluide selon la revendication 7, dans lequel le corps perméable au fluide comporte une ou plusieurs couvertures couvrant au moins une partie de la fente lorsque le conduit est au moins en partie positionné dans la fente.

9. Dispositif selon la revendication 7 dans lequel la fente est en forme de I et s'étend à partir de la deuxième surface du corps perméable au fluide vers une profondeur dans le corps perméable au fluide.

10. Dispositif selon la revendication 7 dans lequel la fente est en forme de L.

11. Dispositif selon la revendication 7 dans lequel la fente est en forme de T avec une première partie (204) sensiblement parallèle à la première surface et deuxième surface du corps perméable au fluide et une deuxième partie (206) qui s'étend à partir de la deuxième surface pour permettre au conduit d'accéder à la première partie de la fente.

12. Dispositif selon l'une quelconque des revendications 7 à 11 dans lequel la fente comporte un trou ou canal complémentaire du conduit.

13. Dispositif selon l'une quelconque des revendications précédentes et comportant un pli ou une couture qui permet à la barrière imperméable au fluide d'obtenir facilement une configuration à biais double pour un positionnement plus fiable du dispositif de collecte de fluide entre les jambes et contre l'urètre d'une utilisatrice, le pli ou la couture étant aligné(e) avec ledit axe.

14. Dispositif selon l'une quelconque des revendications précédentes et comportant des marquages sur la barrière (102) pour aider un professionnel de santé à aligner l'ouverture sur l'urètre ou le pénis d'un porteur du dispositif.

15. Dispositif selon l'une quelconque des revendications précédentes, dans un système qui comporte un contenant de stockage d'urine.
